# EUROPEAN PATENT SPECIFICATION

(11) **EP 1 691 883 B1**
(45) Date of publication and mention of the grant of the patent: **25.07.2012**
(21) Application number: 04818678.7
(22) Date of filing: 08.11.2004
(51) Int. Cl.: A61M 5/145

(54) **DISPENSER FOR PATIENT INFUSION DEVICE**
SPENDER FÜR EIN PATIENTEN-INFUSIONSSYSTEM
DISTRIBUTEUR POUR DISPOSITIFS D'INFUSION POUR PATIENT

(30) Priority: 07.11.2003 US 704291
(43) Date of publication of application: 23.08.2006
(73) Proprietor: Insulet Corporation, Bedford, MA 01730 (US)
(72) Inventor: GARIBOTTO, John T., Marblehead, MA 01945 (US); DIIANNI, Steven, Danvers, MA 01923 (US); FLAHERTY, Christopher J., Topsfield, MA 01983 (US); JAMES, Brian, Reading, MA 01867 (US); SCHMID, Kevin, Bosford, MA 01921 (US); ZELLER, David, Boston, MA 02118 (US)
(74) Representative: Hill, Justin John
(86) International application number: PCT/US2004/037429
(87) International publication number: WO 2005/046756

(56) References cited:
- WO-A-94/15660
- US-A- 4 221 219
- US-A- 5 971 963
- US-B2- 6 564 105

## Description

### Field of the Invention

The present invention relates generally to medical devices, systems and methods, and more particularly to small, low cost, portable infusion devices and methods that are useable to achieve precise, sophisticated, and programmable flow patterns for the delivery of therapeutic liquids such as insulin to a mammalian patient. Even more particularly, the present invention is directed to a dispenser for a fluid delivery device that utilizes a shape memory element.

### Background of the Invention

Today, there are numerous diseases and other physical ailments that are treated by various medicines including pharmaceuticals, nutritional formulas, biologically derived or active agents, hormonal and gene based material and other substances in both solid or liquid form. In the delivery of these medicines, it is often desirable to bypass the digestive system of a mammalian patient to avoid degradation of the active ingredients caused by the catalytic enzymes in the digestive tract and liver. Delivery of a medicine other than by way of the intestines is known as parenteral delivery. Parenteral delivery of various drugs in liquid form is often desired to enhance the effect of the substance being delivered, insuring that the unaltered medicine reaches its intended site at a significant concentration. Also, undesired side effects associated with other routes of delivery, such as systemic toxicity, can potentially be avoided.

Often, a medicine may only be available in a liquid form, or the liquid version may have desirable characteristics that cannot be achieved with solid or pill form. Delivery of liquid medicines may best be accomplished by infusing directly into the cardiovascular system via veins or arteries, into the subcutaneous tissue or directly into organs, tumors, cavities, bones or other site specific locations within the body.

Parenteral delivery of liquid medicines into the body is often accomplished by administering bolus injections using a needle and reservoir, or continuously by gravity driven dispensers or transdermal patch technologies. Bolus injections often imperfectly match the clinical needs of the patient, and usually require larger individual doses than are desired at the specific time they are given. Continuous delivery of medicine through gravity feed systems compromise the patient's mobility and lifestyle, and limit the therapy to simplistic flow rates and profiles. Transdermal patches have special requirements of the medicine being delivered, particularly as it relates to the molecular structure, and similar to gravity feed systems, the control of the drug administration is severely limited.

Ambulatory infusion pumps have been developed for delivering liquid medicaments to a patient. These infusion devices have the ability to offer sophisticated fluid delivery profiles accomplishing bolus requirements, continuous infusion and variable flow rate delivery. These infusion capabilities usually result in better efficacy of the drug and therapy and less toxicity to the patient's system. An example of a use of an ambulatory infusion pump is for the delivery of insulin for the treatment of diabetes mellitus. These pumps can deliver insulin on a continuous basal basis as well as a bolus basis as is disclosed in U:S. Patent 4,498,843 to Schneider et al.

The ambulatory pumps often work with a reservoir to contain the liquid medicine, such as a cartridge, a syringe or an IV bag, and use electro-mechanical pumping or metering technology to deliver the medication to the patient via tubing from the infusion device to a needle that is inserted transcutaneously, or through the skin of the patient. The devices allow control and programming via electromechanical buttons or switches located on the housing of the device, and accessed by the patient or clinician. The devices include visual feedback via text or graphic screens, such as liquid crystal displays known as LCD's, and may include alert or warning lights and audio or vibration signals and alarms. The device can be worn in a harness or pocket or strapped to the body of the patient.

Currently available ambulatory infusion devices are expensive, difficult to program and prepare for infusion, and tend to be bulky, heavy and very fragile. Filling these devices can be difficult and require the patient to carry both the intended medication as well as filling accessories. The devices require specialized care, maintenance, and cleaning to assure proper functionality and safety for their intended long term use. Due to the high cost of existing devices, healthcare providers limit the patient populations approved to use the devices and therapies for which the devices can be used.

Clearly, therefore, there was a need for a programmable and adjustable infusion system that is precise and reliable and can offer clinicians and patients a small, low cost, light- weight, easy-to-use alternative for parenteral delivery of liquid medicines.

In response, the applicant of the present application provided a small, low cost, light-weight, easy-to-use device for delivering liquid medicines to a patient. The device, which is described in detail in co-pending U. S. application serial No. 09/943,992, filed on August 31, 2001, includes an exit port, a dispenser for causing fluid from a reservoir to flow to the exit port, a local processor programmed to cause a flow of fluid to the exit port based on flow instructions from a separate, remote control device, and a wireless receiver connected to the local processor for receiving the flow instructions. To reduce the size, complexity and costs of the device, the device is provided with a housing that is free of user input components, such as a keypad, for providing flow instructions to the local processor.

What are still desired are new and improved components, such as dispensers and reservoirs, for a device for delivering fluid to a patient. Preferably, the components will be simple in design, and relatively compact, lightweight, easy to manufacture and inexpensive, such that the resulting fluid delivery device can be effective, yet inexpensive and disposable.

US Patent No: 4,221,219 discloses an implantable infusion apparatus having a main infusate reservoir and a smaller volume auxiliary infusate reservoir interconnected by a passage.

US Patent No: 6,564,105 discloses an implanted medical device and handheld communication device which communicate with one another via telemetry.

International Patent Publication No: 94/15660 discloses a powered-plunger infusion device comprising a drive system having at least one geared motor to drive a feed screw system.

US 5,971,963 discloses a portable automatic syringe device having a configuration including a separable rotating shaft adapted to provide a drive force to a piston included in the automatic syringe device.

### Summary of the Invention

There is provided a device for delivering fluid to a patient as set out in Claims 1 and 4.

The present invention provides a device for delivering fluid, such as insulin for example, to a patient. The device includes an exit port assembly, a reservoir including a side wall extending towards an outlet connected to the exit port assembly, and a threaded lead screw received in the reservoir and extending towards the outlet of the reservoir. A plunger is secured to the lead screw and has an outer periphery linearly slideable along the side wall of the reservoir. The plunger and the lead screw are operatively arranged such that rotation of the lead screw in a first direction causes the plunger to slide along the side wall towards the outlet of the reservoir, which in turn causes fluid within the reservoir to be dispensed to the exit port assembly. The device also includes a dispenser having a shape memory element having a changeable length decreasing from an uncharged length to a charged length when at least one charge is applied to the shape memory element. The shape memory element has a first end secured to-the lead screw, a portion of the elongated shape memory element wrapped around the lead screw, and a second end fixed with respect to the lead screw, such that the changeable length of the shape memory element decreasing from an uncharged length to a charged length causes rotation of the lead screw in the first direction. The use of a shape memory element helps provide a dispenser that is simple in design, and relatively compact, lightweight, and easy to manufacture.

The present invention, therefore, provides a device for delivering fluid to a patient including new and improved components, such as dispensers and reservoirs. The components are simple in design, and relatively compact, lightweight, easy to manufacture and inexpensive, such that the resulting fluid delivery device is also relatively compact, lightweight, easy to manufacture and inexpensive such that the device can be inexpensive and disposable. In particular, the new and improved components of the present invention advantageously use shape memory elements to reduce complexity and costs.

These aspects of the invention together with additional features and advantages thereof may best be understood by reference to the following detailed descriptions and examples taken in connection with the accompanying illustrated drawings.

### Brief Description of the Drawings

Fig. 1 is a perspective view of a first exemplary embodiment of a fluid delivery device constructed in accordance with the present invention and shown secured on a patient, and a remote control device for use with the fluid delivery device (the remote control device being enlarged with respect to the patient and the fluid delivery device for purposes of illustration);
Fig. 2 is a sectional side view of the fluid delivery device of Fig. 1;
Fig. 3 is a sectional side view of an exemplary embodiment of a reservoir, a plunger and a lead screw of the fluid delivery device of Fig. 1, and an exemplary embodiment of a dispenser constructed in accordance with the present invention for turning the lead screw;
Fig. 4 is an enlarged sectional view of the plunger and the lead screw of the fluid delivery device of Fig. 1;
Fig. 5 is a sectional view of the reservoir, the plunger and the lead screw of the fluid delivery device of Fig. 1 taken along line 5--5 of Fig. 3;
Fig. 6 is an end elevation view, partially in section, of the dispenser for turning the lead screw of the fluid delivery device of Fig. 1;
Fig. 7 is a first end elevation view, partially in section, of another exemplary embodiment of a dispenser constructed in accordance with the present invention for turning the lead screw of the fluid delivery device of Fig. 1;
Figs. 8 and 9 are schematic second end elevation views illustrating operation of the dispenser of Fig. 7;
Figs. 10 through 12 are schematic end elevation views, partially in section, illustrating operation of an additional exemplary embodiment of a dispenser constructed in accordance with the present invention for turning the lead screw of the fluid delivery device of Fig. 1;
Figs. 13 through 15 are schematic end elevation views, partially in section, illustrating operation of a further exemplary embodiment of a dispenser constructed in accordance with the present invention for turning the lead screw of the fluid delivery device of Fig. 1;
Figs. 16 through 19 are side elevation views, partially in section, illustrating operation of still another exemplary embodiment of a dispenser constructed in accordance with the present invention for turning the lead screw of the fluid delivery device of Fig. 1; and
Figs. 20 and 21 are top plan views, partially in section, of exemplary embodiment of an auxiliary component constructed in accordance with the present invention, illustrating actuation of the component;
Fig. 22 is a top plan sectional view of another exemplary embodiment of a fluid delivery device constructed in accordance with the present invention;
Figs. 23 and 24 are enlarged views of the portion of the fluid delivery device contained in circle "23 & 24" of Fig. 22 illustrating operation of a plunger of the device;
Fig. 25 is a side elevation view, partially in section, of still other exemplary embodiments of a dispenser and a reservoir constructed in accordance with the present invention for use as part of a fluid delivery device, such as the fluid delivery device of Fig. 1;
Fig. 26 is a schematic side view of an exemplary embodiment of a portion of another exemplary embodiment of a dispenser constructed in accordance with the present invention;
Fig. 27 is a section view of a portion of the dispenser of Fig. 26 taken along line 27--27 of Fig. 26;
Fig. 28 is a schematic side view of a portion of an additional exemplary embodiment of dispenser constructed in accordance with the present invention;
Fig. 29 is a schematic top view of a portion of a further exemplary embodiment of dispenser constructed in accordance with the present invention;
Figs. 30 and 31 are schematic top views of a portion of still another exemplary embodiment of dispenser constructed in accordance with the present invention; and
Fig. 32 is a schematic end view of a portion of an additional exemplary embodiment of dispenser constructed in accordance with the present invention; and
Figs. 33-35 are schematic views of a portion of an exemplary embodiment of dispenser constructed in accordance with the present invention.
Like reference characters designate identical or corresponding components and units throughout the several views.

### Detailed Description of Exemplary Embodiments

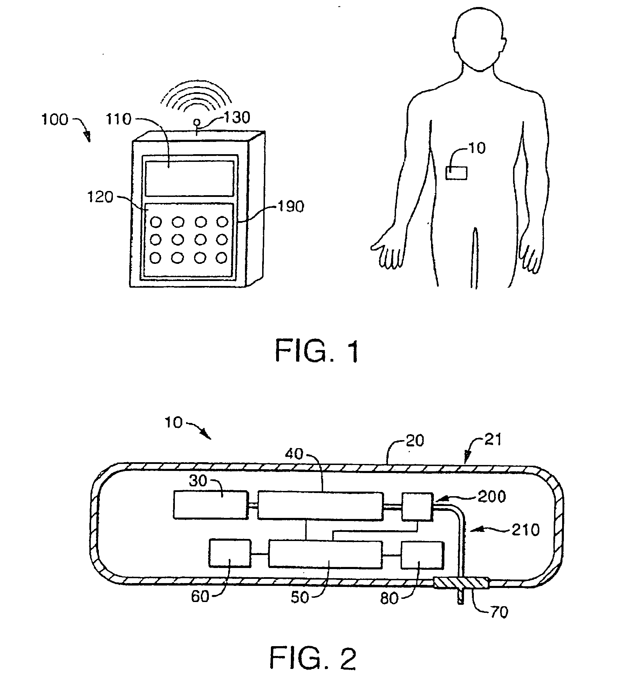

Referring first to Fig. 2, there is illustrated an exemplary embodiment of a fluid delivery device 10 including a dispenser 40 constructed in accordance with the present invention. The dispenser 40 causes fluid flow between a reservoir 30 and an exit port assembly 70 during operation of the device 10. In general, shape memory elements are utilized in accordance with the present invention to provide effective, yet simple and inexpensive dispensers for fluid delivery devices.

The fluid delivery device 10 of Fig. 2 can be used for the delivery of fluids to a person or animal. The types of liquids that can be delivered by the fluid delivery device 10 include, but are not limited to, insulin, antibiotics, nutritional fluids, total parenteral nutrition or TPN, analgesics, morphine, hormones or hormonal drugs, gene therapy drugs, anticoagulants, analgesics, cardiovascular medications, AZT or chemotherapeutics. The types of medical conditions that the fluid delivery device 10 might be used to treat include, but are not limited to, diabetes, cardiovascular disease, pain, chronic pain, cancer, AIDS, neurological diseases, Alzheimer's Disease, ALS, Hepatitis, Parkinson's Disease or spasticity. In addition, it should be understood that the dispenser 40 according to the present invention can be used with fluid delivery devices other than those used for the delivery of fluids to persons or animals.

The fluid delivery device 10 also includes a processor or electronic microcontroller (hereinafter referred to as the "local" processor) 50 connected to the dispenser 40. The local processor 50 is programmed to cause a flow of fluid to the exit port assembly 70 based on flow instructions from a separate, remote control device 100, an example of which is shown in Fig. 1. Referring also to Fig. 2, the fluid delivery device 10 further includes a wireless receiver 60 connected to the local processor 50 for receiving the flow instructions from the separate, remote control device 100 and delivering the flow instructions to the local processor. The device 10 also includes a housing 20 containing the exit port assembly 70, the reservoir 30, the dispenser 40, the local processor 50 and the wireless receiver 60.

As shown, the housing 20 of the fluid delivery device 10 is free of user input components for providing flow instructions to the local processor 50, such as electromechanical switches or buttons on an outer surface 21 of the housing, or interfaces otherwise accessible to a user to adjust the programmed flow rate through the local processor 50. The lack of user input components allows the size, complexity and costs of the device 10 to be substantially reduced so that the device 10 lends itself to being small and disposable in nature. Examples of such devices are disclosed in co-pending U.S. patent application serial number 09/943,992, filed on August 31, 2001 (Atty. Docket No. INSL-110), and entitled DEVICES, SYSTEMS AND METHODS FOR PATIENT INFUSION,

In order to program, adjust the programming of, or otherwise communicate user inputs to the local processor 50, the fluid delivery device 10 includes the wireless communication element, or receiver 60 for receiving the user inputs from the separate, remote control device 100 of Fig. 1. Signals can be sent via a communication element (not shown) of the remote control device 100, which can include or be connected to an antenna 130, shown in Fig. 1 as being external to the device 100.

The remote control device 100 has user input components, including an array of electromechanical switches, such as the membrane keypad 120 shown. The control device 100 also includes user output components, including a visual display, such as a liquid crystal display (LCD) 110. Alternatively, the control device can be provided with a touch screen for both user input and output. Although not shown in Fig. 1, the remote control device 100 has its own processor (hereinafter referred to as the "remote" processor) connected to the membrane keypad 120 and the LCD 110. The remote processor receives the user inputs from the membrane keypad 120 and provides "flow" instructions for transmission to the fluid delivery device 10, and provides information to the LCD 110. Since the remote control device 100 also includes a visual display 110, the fluid delivery device 10 can be void of an information screen, further reducing the size, complexity and costs of the device 10.

The communication element 60 of the device 10 preferably receives electronic communication from the remote control device 100 using radio frequency or other wireless communication standards and protocols. In a preferred embodiment, the communication element 60 is a two-way communication element, including a receiver and a transmitter, for allowing the fluid delivery device 10 to send information back to the remote control device 100. In such an embodiment, the remote control device 100 also includes an integral communication element comprising a receiver and a transmitter, for allowing the remote control device 100 to receive the information sent by the fluid delivery device 10.

The local processor 50 of the device 10 contains all the computer programs and electronic circuitry needed to allow a user to program the desired flow patterns and adjust the program as necessary. Such circuitry can include one or more microprocessors, digital and analog integrated circuits, resistors, capacitors, transistors and other semiconductors and other electronic components known to those skilled in the art. The local processor 50 also includes programming, electronic circuitry and memory to properly activate the dispenser 40 at the needed time intervals.

In the exemplary embodiment of Fig. 2, the device 10 includes a power supply 80, such as a battery or capacitor, for supplying power to the local processor 50. The power supply 80 is preferably integrated into the fluid delivery device 10, but can be provided as replaceable, e.g., a replaceable battery.

Although not shown, the device 10 can include sensors or transducers such as a reservoir volume transducer or a reservoir pressure transducer, for transmitting information to the local processor 50 to indicate how and when to activate the dispenser 40, or to indicate other parameters determining flow, pump flow path prime condition, blockage in flow path, contact sensors, rotary motion or other motion indicators, as well as conditions such as the reservoir 30 being empty or leaking, or the dispensing of too much or too little fluid from the reservoir, etc.

The volume of the reservoir 30 is chosen to best suit the therapeutic application of the fluid delivery device 10 impacted by such factors as available concentrations of medicinal fluids to be delivered, acceptable times between refills or disposal of the fluid delivery device 10, size constraints and other factors. The reservoir 30 may be prefilled by the device manufacturer or a cooperating drug manufacturer, or may include external filling means, such as a fill port having needle insertion septum or a Luer connector, for example. In addition, the device 10 can be provided with a removable reservoir.

The exit port assembly 70 can include elements to penetrate the skin of the patient, such that the entire volume of the flow path 210 of the fluid delivery device 10 is predetermined. For example, a needle-connection tubing terminating in a skin penetrating cannula (not shown) can be provided as an integral part of the exit port assembly 70, with the skin penetrating cannula comprising a rigid member, such as a needle. The exit port assembly 70 can further be provided with injection means, such as a spring driven mechanism, to assist in penetrating the skin with the skin penetrating cannula. For example, if the cannula is a flexible tube, a rigid penetrator within the lumen of the tube can be driven through the skin by the injection means and then withdrawn, leaving the soft cannula in place in the subcutaneous tissue of the patient or other internal site. The injection means may be integral to the device 10, or removable soon after transcutaneous penetration.

Alternatively, the exit port assembly 70 can be adapted to connect, with a Luer connector for example, to a separate, standard infusion device that includes a skin penetrating cannula. In any event, the exit port assembly 70 can also be provided with a removable plug (not shown) for preventing leakage during storage and shipment if pre-filled, and during priming if filled by user, and prior to use. It should be understood that, as used herein, the term "flow path" is meant to include all portions of the fluid delivery device 10 that contain therapeutic fluid for delivery to a patient, e.g., all portions between the fill port of the reservoir to the tip of the needle of the exit port assembly.

Although not shown, the device 10 can also be provided with an adhesive layer on the outer surface of the housing 20 for securing the device 10 directly to the skin of a patient. The adhesive layer is preferably provided in a continuous ring encircling the exit port assembly 70 in order to provide a protective seal around the penetrated skin. The housing 20 can be made from flexible material, or can be provided with flexible hinged sections that allow the fluid delivery device 10 to flex during patient movement to prevent detachment and aid in patient comfort.

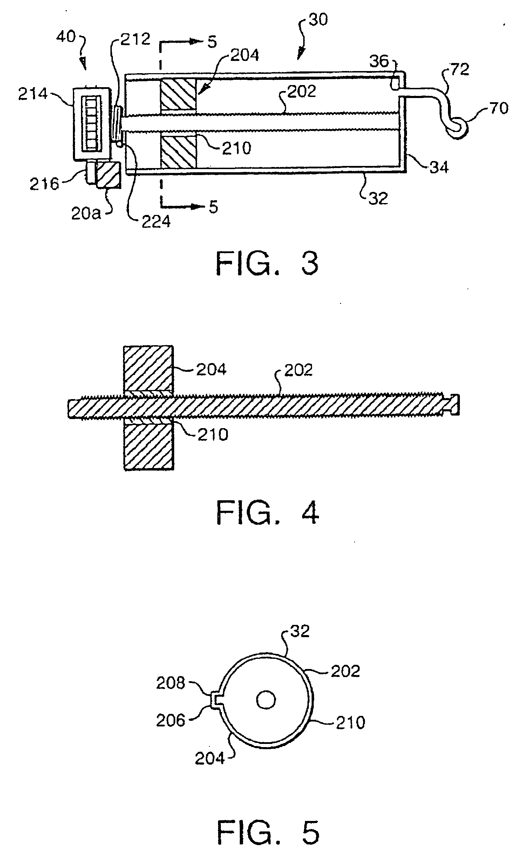

Referring to Figs. 3 through 19 and 22 through 25, the present disclosure provides various combinations of dispensers and reservoirs for use with the fluid delivery device 10 of Figs. 1 and 2. The dispensers and the reservoirs are small and simple in design, and inexpensive and easy to manufacture, in order to further reduce the size, complexity and costs of the fluid delivery device 10, such that the device 10 continues to lend itself to being small and disposable in nature. In general, the device 10 is provided with non-pressurized reservoirs, and the dispensers are adapted to cause flow from the reservoirs. The dispensers are controlled by the local processor 50, which includes electronic programming, controls, and circuitry to allow sophisticated fluid delivery programming and control of the dispensers.

Referring to Figs. 3 through 5, the reservoir 30 is provided with a side wall 32 extending between an open end and an end wall 34 of the reservoir. The end wall 34 includes an outlet 36 connected through a lumen 72 to the exit port assembly 70 of the device 10. The reservoir 30 also includes a threaded lead screw 202 mounted for rotation within the reservoir 30, and a plunger 204 threadedly received on the lead screw. The lead screw 202 is positioned coaxial with the side wall 32 and extends to the end wall 34 of the reservoir 30. The plunger 204 and the reservoir 30 are adapted such that a seal is formed between the plunger 204 and the lead screw 202 and the plunger 204 and the side wall 32 of the reservoir, so that movement of the plunger 204 towards the end wall 34 of the reservoir 30 will force fluid through the outlet 36 to the exit port assembly 70.

The plunger 204 is prevented from rotation with respect to the side wall 32 so that, when the screw 202 is turned with respect to the plunger 204, the plunger is caused to move along the screw 202 within the reservoir 30. In the embodiment shown in Fig. 5, the reservoir 30 and the plunger 204 are provided with circular cross-sections, but the plunger 204 has at least one protrusion 206 radially extending into a channel 208 in the side wall 32 of the reservoir 30 to prevent rotation of the plunger. Alternatively, the plunger 204 can be provided with at least one channel and the side wall 32 of the reservoir 30 can be provided with at least one protrusion extending along its length and received within the channel of the plunger to prevent rotation of the plunger. In addition, the reservoir 30 and the plunger 204 can alternatively be provided with corresponding non-circular cross-sections, such as oval, square or rectangular, to prevent rotation of the plunger 204 with respect to the side wall, without the use of a protrusion and a channel. Such non-circular cross-sections can also include simply providing the side wall and the plunger with mating flat portions in otherwise circular cross-sections.

An advantage of the reservoir 30 of Figs. 3 through 5 is that it utilizes an integrated lead screw 202 that extends to the end wall 34 of the reservoir, and thus has an overall length reduction as compared to a syringe having a reservoir with a separate sliding plunger and lead screw extending out of the open end of the reservoir. Another advantage of the reservoir 30 is that the plunger 204 and the internal lead screw 202 are entirely contained within the reservoir 30, and do not require mechanisms or procedures for pulling the plunger back to remove a used syringe or re-load a full syringe. Such mechanisms or procedures can increase the costs, complexity, and size and weight, and decrease the reliability of a fluid delivery device. Thus, the reservoir 30 Figs. 3 through 5 advantageously does not need such mechanisms or procedures.

In order to further reduce the cost of the reservoir 30, the lead screw 202 and the plunger 204 are preferably made from an inexpensive material. The lead screw 202 is made of a rigid material such as a metal, such as stainless steel, or a plastic, such as polyethylene or polypropylene. The side wall 32 and the end wall 34 of the reservoir are preferably made from a rigid material such as a suitable metal (e.g., stainless steel) or plastic. The plunger 204, however, is made of a flexible material, such as a silicone elastomer or rubber, and provided with a rigid insert 210 made of metal or plastic for engaging the threads of the lead screw 202. Since the device is preferably disposable, preventing thread wear between the lead screw 202 and the plunger 204 is not necessary, thereby allowing the use of less expensive materials and lower tolerances in the manufacture and assembly of the lead screw 202 and the plunger 204.

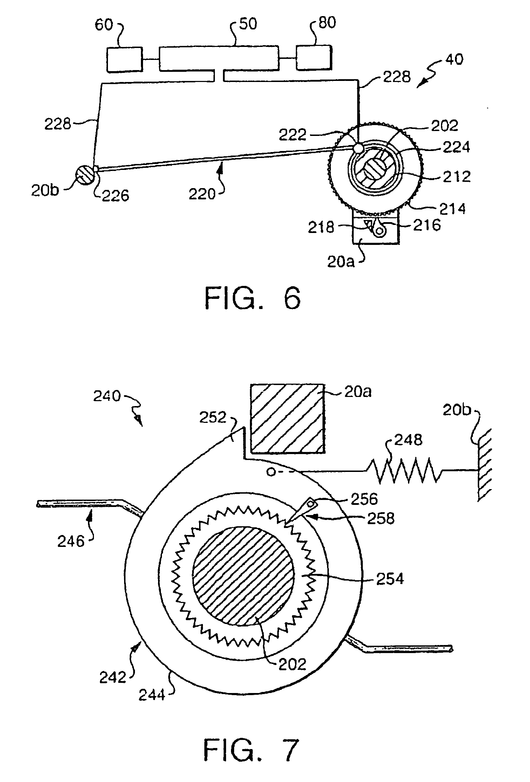

Referring also to Fig. 6, the dispenser 40 causes fluid flow by turning the lead screw 202 of the reservoir 30. In the embodiment of Figs. 3 and 6, the dispenser 40 includes a hub 212 coaxially fixed to the lead screw 202, such that rotation of the hub causes rotation of the lead screw. Coaxially secured to the hub 212 is a gear 214 having radially extending teeth. The lead screw 202 and the plunger 204 are adapted such that rotation of the lead screw in a first direction, which is counter-clockwise as shown in Fig. 6, causes movement of the plunger 204 towards the end wall 34 of the reservoir 30 to force fluid through the outlet 36 to the exit port assembly 70. The dispenser also includes a pawl 216 pivotally mounted on a fixed member 20a of the housing 20 of the fluid delivery device 10. The pawl 216 engages the teeth of the gear 214 and is arranged with a backstop 218 to prevent rotation of the gear 214, the hub 212 and the lead screw 202 in a second direction, which is clockwise as shown in Fig. 6.

The exemplary embodiment of the dispenser 40 of the present invention also includes a shape memory element 220 made of a shape memory material. The application of an electrical current to a shape memory material results in molecular and crystalline restructuring of the shape memory material. If the shape memory material is in the shape of an elongated wire, for example, as the shape memory element 220 preferably is, this restructuring causes a decrease in length. Nitinol, a well-known alloy of nickel and titanium, is an example of such a so-called shape memory material and is preferred for use as the shape memory element 220.

As shown best in Fig. 6, a first end 222 of the shape memory element 220 is secured to the hub 212, a portion 224 of the shape memory element 220 is wrapped around the hub 212, and a second end 226 of the shape memory element 220 is secured to a fixed internal portion of the housing 20 of the fluid delivery device 10. The dispenser 40 includes wires 228 connecting the opposite ends 222, 226 of the shape memory element 220 to the processor 50 of the fluid delivery device. When a charge is applied to the shape memory element 220 through the wires 228, the length of the shape memory element 220 decreases from an uncharged length to a charged length. The decrease in length occurs with a force that is sufficient to rotate the hub 212 and the lead screw 202 in the first direction to advance the plunger 204. The dispenser 40 does not include means for pulling the shape memory element 220 back to its original length upon the charge being removed from the element.

Although not shown, the processor 50 can include capacitors for storing a charge received from the power source 80. The fluid delivery device 10 is calibrated so that a single charge from the processor 50 causes the dispensing of a predetermine volume of fluid, called pulse volume (PV), from the reservoir 30. In this manner, a desired volume to be delivered by the fluid delivery device 10 is dispensed by the release of multiple charges over a predetermined period. PV's delivered by infusion devices are typically chosen to be small relative to what would be considered a clinically significant volume. For insulin applications at a concentration of one hundred units per microliter (100 units/ml), a PV of less than two microliters, and typically a half of a microliter, is appropriate. If the fluid delivery device 10 is programmed via the remote control device 100 to deliver two units an hour, the processor 50 will deliver forty charges an hour, or a charge every ninety seconds, to the shape memory element 220. Other drugs or concentrations may permit a much larger PV. Various flow rates are achieved by adjusting the time between charges. To give a fixed volume or bolus, multiple charges are given in rapid succession until the bolus volume is reached.

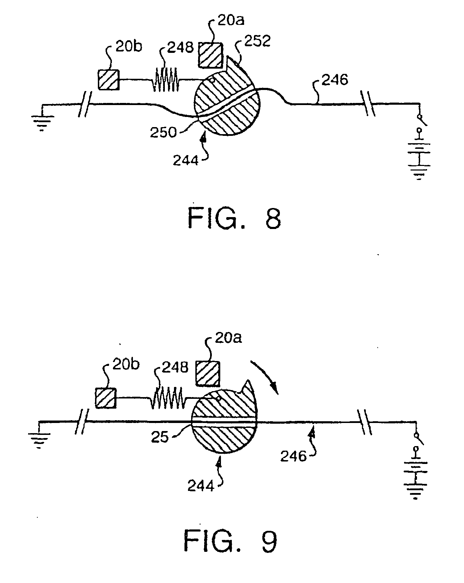

Another exemplary embodiment of a dispenser 240 constructed in accordance with the present invention is shown in Figs. 7 through 9. The dispenser 240 includes a ratchet mechanism 242 secured to the lead screw 202 and including a wheel 244 arranged such that rotation of the wheel in a first direction (which is clockwise as shown in Fig. 7 and counter-clockwise as shown in Figs. 8 and 9) causes rotation of the lead screw 202 in the first direction, while rotation of the wheel 244 in a second direction (which is counter-clockwise as shown in Fig. 7 and clockwise as shown in Figs. 8 and 9) causes no rotation of the lead screw 202.

The dispenser 240 also includes an elongated shape memory elements 246 operatively connected to the wheel 244 of the ratchet mechanism 242 such that the changeable length of the shape memory element 246 decreasing from an uncharged length to a charged length causes rotation of the wheel 244 in one of the first direction and the second direction. An actuation element 248 is secured to the wheel 244 for moving the wheel in the other of the first direction and the second direction.

In the embodiment shown in Figs. 7 through 9, the actuation element comprises a spring 248. The spring 248 biases the wheel 244 in the second direction, while the changeable length of the shape memory element 246 decreasing from an uncharged length to a charged length overcomes the biasing force of the spring 248 and causes rotation of the wheel 244 in the first direction. As shown best in Figs. 8 and 9, the spring is a helical tension spring 248 that expands upon the shape memory element 246 decreasing from an uncharged length to a charged length, and contracts to increase the shape memory element 246 from a charged length to an uncharged length.

The shape memory element 246 comprises an elongated wire that extends through a traverse passage 250 in the wheel 244, and the spring 248 normally biases the wheel such that an uncharged length of the shape memory element is bent, as shown in Fig. 8. The shape memory element 246 decreasing from an uncharged length to a charged length straightens the charged length of the shape memory element and rotates the wheel 244 in the first direction against the bias of the spring 248, as shown in Fig. 9. When the charge is removed, the spring 248 biases the wheel 244, increases the length of the shape memory element 246 to the uncharged length, and bends the uncharged length of the shape memory element, as shown in Fig. 8.

The dispenser 240 preferably includes means for limiting rotation of the wheel 244 in the first direction. In particular, the wheel 244 includes a radially extending tooth 252 positioned to contact a first fixed member 20a of the device housing and limit rotation of the wheel 244 in the first direction. The spring 248 extends between the wheel 244 and a second fixed member 20d of the device housing and normally pulls the tooth 252 against the first fixed member 20a.

As shown best in Fig. 7, the ratchet mechanism 242 further includes a gear 252 secured to the lead screw 202 coaxially within the wheel 244 and having teeth extending radially outwardly towards the wheel. A pawl 256 is pivotally mounted on the wheel 244 and extends radially inwardly from the wheel and engages the teeth of the gear 252. A backstop 258 limits pivotal motion of the pawl 256 to prevent relative rotation between the wheel 244 and the gear 252 during rotation of the wheel in the first direction, and allow relative rotation between the wheel and the gear during rotation of the wheel 244 in the second direction. In this manner rotation of the wheel 244 in the first direction causes rotation of the gear 252 and the lead screw 202 (and advancement of the plunger), while rotation of the wheel 244 in the second direction does not cause rotation of the lead screw 202.

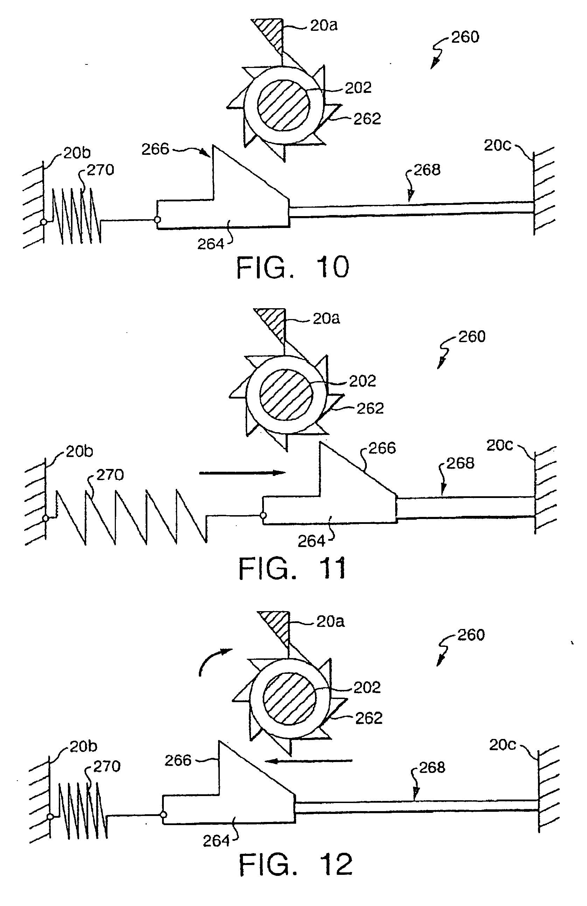

An additional exemplary embodiment of a dispenser 240 constructed in accordance with the present invention is shown in Figs. 10 through 12. The dispenser 240 includes a gear 262 secured to the lead screw 202 and including radially extending teeth positioned to contact a first fixed member 20a of the device housing and prevent rotation of the gear 262 and the lead screw 202 in the second direction (which is counter-clockwise as shown in Figs. 10 through 12). A slide 264 is positioned for linear movement adjacent the gear 262 between a second fixed member 20b and a third fixed member 20c. The slide 264 includes a finger 266 for engaging the teeth of the gear 262, and the finger and the teeth are adapted such that linear movement of the slide 264 past the gear 262 towards the second fixed member 20b, as shown in Fig. 12, causes rotation of the gear 262 in the first direction (which is clockwise as shown in Figs. 10 through 12). The finger 266 and the teeth of the gear 262 are also adapted such that linear movement of the slide 264 past the gear towards the third fixed member 20c, as shown in Fig. 11, causes no rotation of the gear (i.e., the finger and the teeth are shaped to slide over each other as the slide 264 moves past the gear 262 towards the third fixed member 20c).

The dispenser also includes an elongated shape memory element 268 connected between the slide 264 and the third fixed member 20c, such that the changeable length of the shape memory element 268 decreasing from an uncharged length to a charged length causes linear movement of the slide 264 past the gear 262 towards the third fixed member 20c, as shown in Fig. 11. An actuation element 270 is connected between the slide 264 and the second fixed member 20b for causing linear movement of the slide 264 past the gear 262 towards the second fixed member 20b when the shape memory element 268 increases from a charged length to an uncharged length, as shown in Fig. 12. The actuation element 270, therefore, rotates the lead screw 202 in the first direction (and advances the piston in the reservoir to dispense fluid to the exit port assembly).

In the embodiment of Figs. 10 through 12, the actuation element comprises a spring 270. Preferably, the spring is a helical tension (or extension) spring 270 that expands upon the shape memory element 268 decreasing from an uncharged length to a charged length, and contracts to increase the shape memory element 268 from a charged length to an uncharged length.

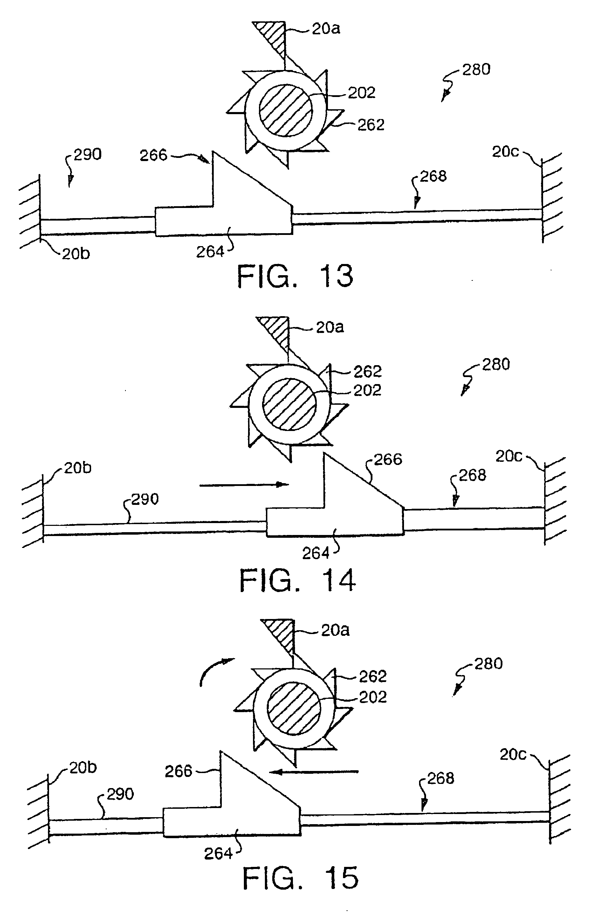

A further exemplary embodiment of a dispenser 280 constructed in accordance with the present invention is shown in Figs. 13 through 15. Operation of the dispenser 280 is similar to operation of the dispenser 260 of Figs. 10 through 12. In addition, elements of the dispenser 280 are similar to elements of the dispenser 260 of Figs. 10 through 12 such that similar elements have the same reference numeral. In the embodiment 280 of Figs. 13 through 15, however, the actuation element comprises a second elongated shape memory element 290. The second shape memory element 290 is connected between the slide 264 and the second fixed member 20b such that the changeable length of the second shape memory element 290 decreasing from an uncharged length to a charged length causes linear movement of the slide 264 past the gear 262 towards the second fixed member 20b. The (first) shape memory element 268 and the second shaped memory element 290 are alternatively charged to cause linear motion of the slide 264 and rotation of the gear 262 and the lead screw 202.

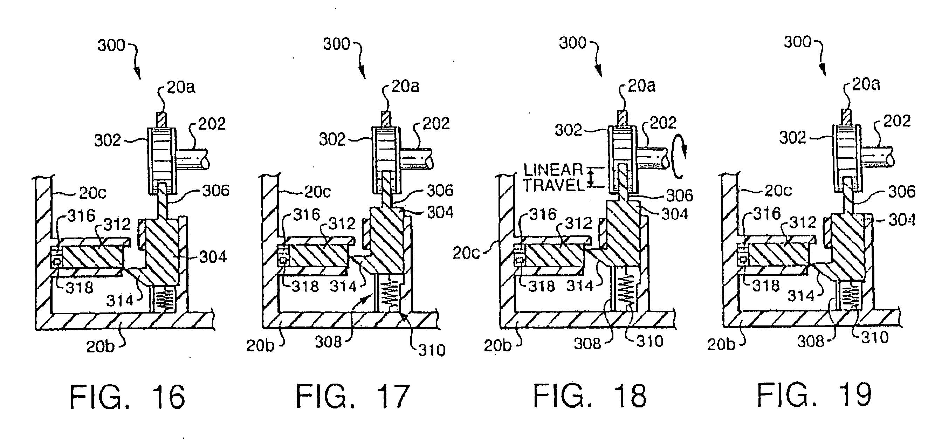

Still another exemplary embodiment of a dispenser 300 constructed in accordance with the present invention is shown in Figs. 16 through 19. The dispenser 300 includes a gear 302 secured to the lead screw 202 and having radially extending teeth positioned to contact a first fixed member 20a of the device housing and prevent rotation of the gear 302 and the lead screw 202 in the second direction. A slide 304 is positioned for linear movement between the gear 302 and a second fixed member 20b. The slide 304 includes a finger 306 for engaging the teeth of the gear 302, and the finger 306 and the teeth are adapted such that linear movement of the slide 304 towards the gear 302, as shown in Figs. 17 and 18, causes rotation of the gear 302 in the first direction while linear movement of the slide 304 towards the second fixed member 20b, as shown in Fig. 19, causes no rotation of the gear 302.

The dispenser 300 of Figs. 16 through 19 also includes an elongated shape memory element 308 connected between the slide 304 and the second fixed member 20b such that the changeable length of the shape memory element 308 decreasing from an uncharged length to a charged length causes linear movement of the slide 304 towards the second fixed member 20b. An actuation element 310 is connected between the slide 304 and the second fixed member 20b for causing linear movement of the slide 304 towards the gear 302 when the shape memory element 308 is uncharged. As shown, the actuation element comprises a helical compression spring 310 that contracts upon the shape memory element 308 decreasing from an uncharged length to a charged length, and expands to increase the shape memory element 308 from a charged length to an uncharged length.

The dispenser 300 further includes a latch 312 movable between the slide 304 and a third fixed member 20c. The latch 312 is adapted and arranged to engage a shoulder 314 of the slide 304, when the latch 312 is moved to the slide 304, to prevent movement of the slide 304 towards the gear 302. A second elongated shape memory element 316 is connected between the latch 312 and the third fixed member 20c such that the changeable length of the second shape memory element 316 decreasing from an uncharged length to a charged length causes movement of the latch 312 towards the third fixed member 20c.

A second actuation element 318 is connected between the latch 312 and the third fixed member 20c for causing movement of the latch 312 towards the slide 304 when the second shape memory element 316 is uncharged. As shown, the second actuation element also comprises a helical compression spring 318 that contracts upon the second shape memory element 316 decreasing from an uncharged length to a charged length, and expands to increase the second shape memory element 316 from a charged length to an uncharged length.

During operation, the second shaped memory element 316 is charged to pull the latch 312 off the shoulder 314 of the slide 304, as shown in Fig. 17, and allow the first spring 310 to bias the slide 304 towards the gear 302 and rotate the gear 302 and the lead screw 202 in the first direction, as shown in Fig. 18. Thus, the first spring 310 actually causes rotation of the lead screw 202 (and advancement of the piston).

Then, as shown in Fig. 19, a charge is applied to the first shape memory element 308 to pull the slide 304 away from the gear 302 and back towards the second fixed member 20b such that the shoulder 314 of the slide 304 falls below the level of the latch 312. The charge is then removed from the second shape memory element 316, such that the second spring 318 is allowed to move the latch 312 towards the slide 304 and over the shoulder 314, as shown in Fig. 16. Thereafter, the charge can be removed from the first shape memory element 308 since the shoulder 314 caught by the latch 312 will prevent the first spring 310 from moving the slide 304 to the gear 302. The steps illustrated in Figs. 16 through 19 are successively repeated (through electrical charges provided by the local processor) to produce pulse volumes of fluid flow from the reservoir.

An end of the shape memory element of any of the above described dispensers can be connected to an auxiliary component of the fluid delivery device 10 for actuating the auxiliary component upon at least a first charge applied to the shape memory element. For example, the auxiliary component can comprise a spring-loaded needle of the exit port assembly and the shape memory element can be arranged to release the spring-loaded needle for insertion into a patient upon first decreasing from an uncharged length to a charged length when a first, or initial, charge is applied to the shape memory element.

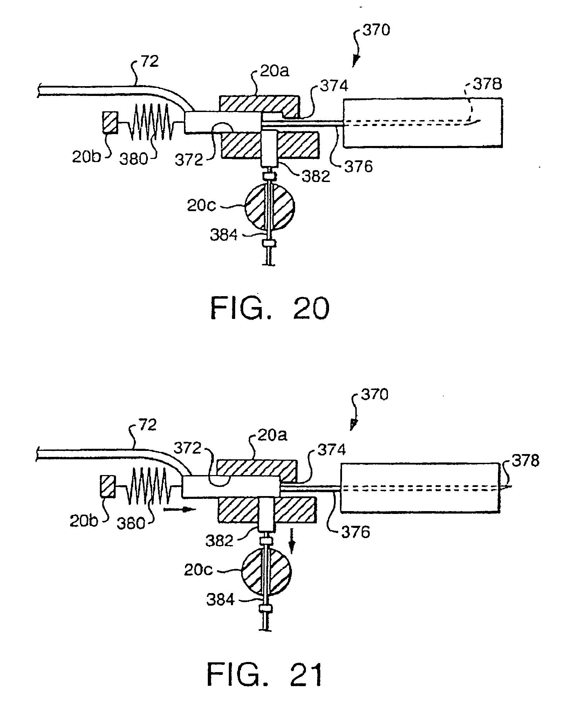

Figs. 20 and 21 show an exemplary embodiment of an auxiliary component 70 constructed in accordance with the present invention for use with the shape memory elements of the dispensers disclosed herein. The auxiliary component is provided in the form of an exit port assembly 70 including a fixed member 20a of the device housing defining a channel 372 having an outlet 374, and a transcutaneous patient access tool 376 received for sliding movement in the channel 372 towards the outlet 374. In the embodiment shown, the transcutaneous patient access tool comprises a rigid needle 376 having a sharpened, hollow end 378.

The exit port assembly 370 also includes an actuation element 380 connected between the tool 376 and a second fixed member 20b for causing sliding movement of the tool towards the outlet 374 of the channel 372. In the embodiment shown, the actuation element comprises a helical compression spring 380 that expands to cause sliding movement of the needle 376 towards the outlet 374 of the channel 372.

A latch 382 is removably positioned within the channel 372 between the needle 376 and the outlet 374 to prevent the spring 380 from moving the needle to the outlet, as shown in Fig. 20. An elongated shape memory element 384 is connected to the latch 382 such that the changeable length of the shape memory element 384 decreasing from an uncharged length to a charged length causes movement of the latch 382 out of the channel 372 and release of the needle 376, as shown in Fig. 21. The elongated shaped memory element 384 preferably comprises the elongated shaped memory element of a dispenser, such as the dispensers disclosed herein, of the fluid delivery device, such that a single elongated shaped memory element is used to operate two components of the device.

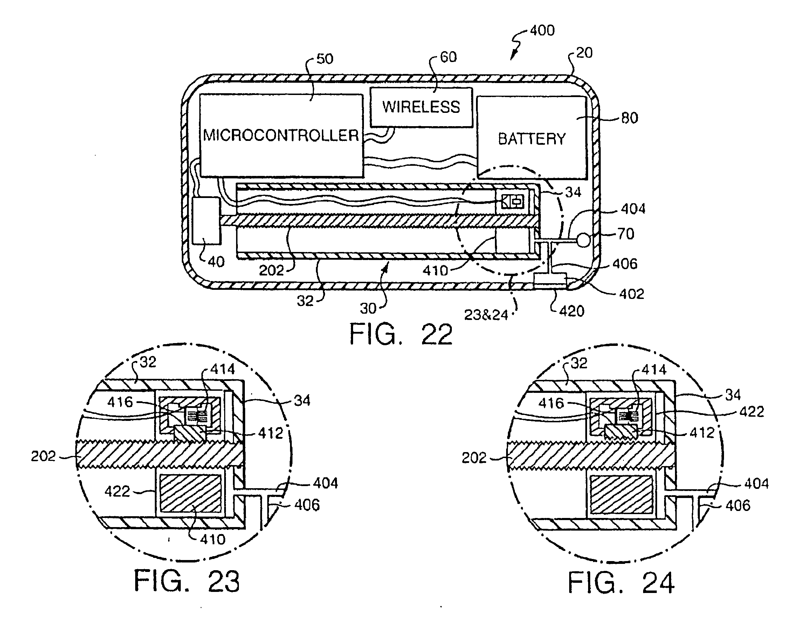

Figs. 22 through 24 show another exemplary embodiment of a fluid delivery device 400 constructed in accordance with the present invention. Operation of the device 400 of Fig. 22 is similar to the operation of the device 10 of Figs. 1 and 2, and similar elements have the same reference numeral.

Referring to Fig. 22, the device 400 includes an exit port assembly 70, a fill port 402, and a reservoir 30 including a side wall 32 extending towards an outlet 404 connected to the exit port assembly 70 and an inlet 406 connected to the fill port 402. A threaded lead screw 202 is received in the reservoir 30 and extends towards the outlet 404 and the inlet 406, generally parallel with the side wall 32, and a plunger 410 has an outer periphery slidably received on the side wall 32 of the reservoir 30 and an inner periphery slidably received on the lead screw 202.

Referring also to Figs. 23 and 24, the plunger 410 is non-rotatable with respect to the side wall 32 and includes an insert 412 having a threaded surface mateable with the threaded lead screw 202, and a spring 414 biasing the threaded surface of the insert 412 against the threaded lead screw 202, as shown in Fig. 23, such that rotating the lead screw 202 in a first direction causes the plunger 410 to slide along the side wall 32 towards the outlet 404 of the reservoir 30. The plunger 410 also includes an elongated shape memory element 416 having a first end secured to the insert 412 and a second end extending radially outwardly from the insert 412 and secured to the plunger 410, such that the changeable length of the shape memory element 416 decreasing from an uncharged length to a charged length pulls the threaded surface of the insert 412 away from the threaded lead screw 202, as shown in Fig. 24.

Thus, when no charge is applied to the shape memory element 416, the insert 412 engages the lead screw 202 as shown in Fig. 23. Applying a charge to the shape memory element 416, however, disengages the insert 412 from the lead screw 202, as shown in Fig. 24, and allows the plunger 410 to slide along the lead screw 202 and away from the inlet 406 of the reservoir 30 during filling of the reservoir through the fill port 402.

The device 400 also includes a dispenser 40 operatively connected to the lead screw 202 for rotating the lead screw to advance the plunger 410 towards the outlet 404 of the reservoir 30. The dispenser can comprise a rotary motor 40 mated to an end of the lead screw 202 and controlled by the local processor 50 of the device 400. As shown, the local processor 50 is also connected to the ends of the shape memory element 416, through wires 418, for controlling the shape memory element by applying or removing a charge to the shape memory element.

In the embodiment shown in Fig. 22, the fill port 402 includes a needle-pierceable septum 420. Although not shown, the device 400 can further include a sensor, such as a pressure switch, connected to the local processor 50 and adapted and arranged to provide a signal upon the presence of a needle in the fill port 402. The local processor 50, in-turn, can be programmed to apply a charge to the shape memory element 416 of the plunger 410 whenever it receives a signal from the fill port sensor. Thus when a needle is positioned in the fill port 402, the plunger insert 412 is disengaged from the lead screw 202 to allow the plunger 410 to slide on the lead screw 202, away from the inlet 406, upon fluid being added to the reservoir 30 through a needle inserted into the fill port 402. Alternatively, the device 400 can be provided with a manual actuator, such as a button for a user to push, for applying a charge to the shape memory element 416 during a filling process.

As shown best in Figs. 23 and 24, the plunger 410 preferably includes an outer layer 422 of resiliently flexible material providing a substantially fluid-tight interface between the outer periphery of the plunger 410 and the side wall 32 of the reservoir 30 and the inner periphery of the plunger 410 and the lead screw 202.

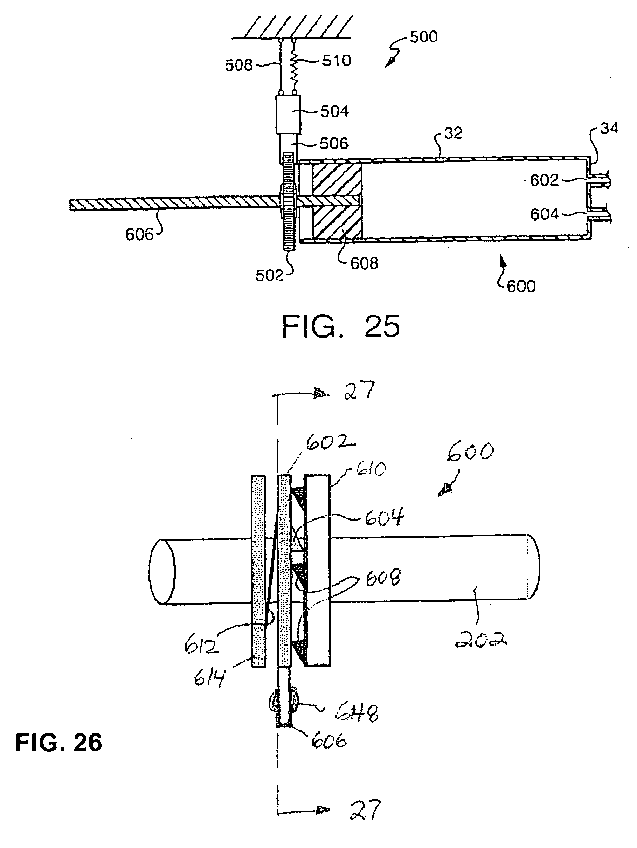

Referring to Fig. 25, another dispenser 500 and reservoir 600 constructed in accordance with the present invention for use with a fluid delivery device, such as the fluid delivery device 10 of Figs. 1 and 2, are shown. The reservoir 600 is provided with a side wall 32 extending between an open end and an end wall 34 of the reservoir. The end wall 34 includes an outlet 602 for connection to the exit port assembly of the device, and an inlet 604 for connection to a fill port of the device. The reservoir 600 also includes a threaded lead screw 606 extending into the reservoir, and a plunger 608 secured to an end of the lead screw. The plunger 608 and the reservoir 600 are adapted such that a seal is formed between the plunger 608 and the lead screw 606 and the plunger and the side wall 32, so that movement of the plunger towards the end wall 34 of the reservoir 600 will force fluid through the outlet 602 to the exit port assembly.

The dispenser 500 causes fluid flow by causing linear movement of the lead screw 606 and the plunger 608 towards the outlet of the reservoir 30. In the embodiment of Fig. 25, the dispenser 500 includes a rotatable gear 502 linearly fixed with respect to the reservoir 600. The gear 502 is coaxially mounted with respect to the lead screw 606, and is threadedly engageable with the lead screw 606, such that rotation of the gear 502 causes linear movement of the lead screw. In particular, the lead screw 606 and the gear 502 are adapted such that rotation of the gear 502 in a first direction causes linear movement of the lead screw 606 and the plunger 608 towards the end wall 34 of the reservoir 600 to force fluid through the outlet 36 to the exit port assembly.

The dispenser 500 of Fig. 25 also includes a slide 504 having a finger 506 for successively engaging teeth of the gear 502, similar to the slide 304 and the finger 306 of the dispenser 300 of Figs. 16 through 19. The dispenser 500 additionally includes a combination of a shape memory element 508 and a spring 510, similar to the shape memory element 308 and the spring 310 of the dispenser 300 of Figs. 16 through 19, for causing the slide 504 and the finger 506 to successively rotate the gear 502 to advance the lead screw 606 and the plunger 608.

Although not shown, the gear 502 of Fig. 25 is configured similar to the plunger 410 of Figs. 22 through 24 so that the gear 502 can be released from the lead screw 606 to allow the lead screw 606 and the plunger 608 to be linearly moved away from the inlet 604 of the reservoir 600 during filling of the reservoir. In particular, the gear 502 includes an insert having a threaded surface mateable with the threaded lead screw 606, and a spring biasing the threaded surface of the insert against the threaded lead screw 606. The gear 502 also includes an elongated shape memory element having a first end secured to the insert and a second end extending radially outwardly from the insert and secured to the gear 502, such that the changeable length of the shape memory element decreasing from an uncharged length to a charged length pulls the threaded surface of the insert away from the threaded lead screw 606. Thus, when no charge is applied to the shape memory element, the insert engages the lead screw 606. Applying a charge to the shape memory element, however, disengages the insert of the gear 502 from the lead screw 606 and allows the lead screw 606 to move linearly with respect to the gear 502 during filling of the reservoir through the inlet.

Referring to Figs. 26, 27 and 32, there are shown further exemplary embodiments of dispensers 600, 700, 800, 900, 1000 constructed in accordance with the present invention for use with a fluid delivery device, such as the fluid delivery device 10 of Figs. 1 and 2. These embodiments provide additional examples of transmissions for allowing the linear actuators to engage and cause rotation of the lead screw 202. In general, the transmissions each include frictional engagement surfaces or elements coupled to the lead screw 202 such that the frictional engagement surface is immovable relative to the lead screw, and engagement means for selectively engaging the frictional engagement surface such that the engagement means engages the frictional engagement surface when the engagement means is moved in a first direction, but does not engage the frictional engagement surface when the engagement means is moved in a second direction. Preferably, the engagement means are made from a stamped piece of metal or other resilient material. One benefit of using stamped pieces of material is that the engagement means are easier and less expensive to manufacture, especially in mass-production. The engagement means are also arranged with respect to the frictional engagement surfaces such that precision engagement, or meshing, is not required between the engagement means and the frictional engagement surfaces, to thereby further simplify manufacturing of the fluid delivery device.

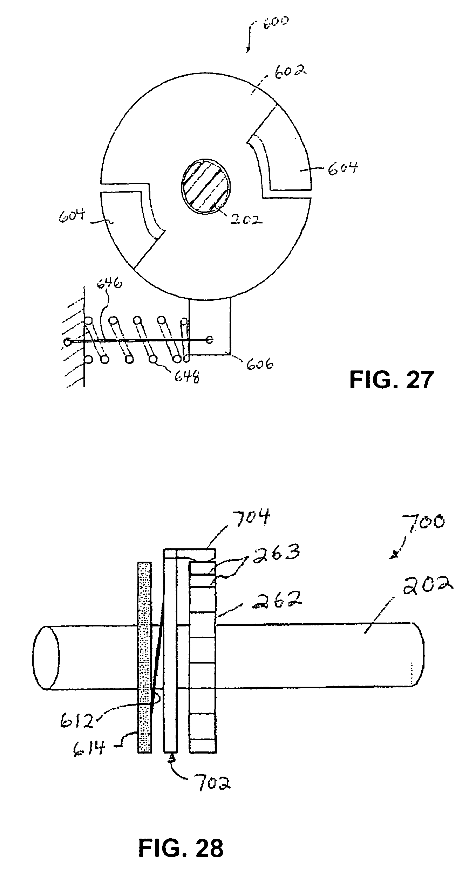

The dispenser 600 shown in Figs. 26 and 27 includes engagement means in the form of a disk 602 rotatably and coaxially mounted for independent rotation on the lead screw 202. The disk 602, which is substantially planar, has at least one finger 604 that projects outwardly from a face of the disk 602 and is adapted to engage a frictional engagement surface comprising teeth 608 extending outwardly from a face of a gear 610. The gear 610 is mounted on the lead screw 202 such that rotation of the gear 610 causes rotation of the lead screw 202.

The finger 604 of the disk 602 is biased towards the gear 610 and includes a flat surface that engages flat surfaces of the teeth 608 so that, when the disk 602 is rotated in a first direction, the finger 604 engages the teeth 608 of the gear 610 and causes the gear and the lead screw 202 to rotate in the first direction. The finger 604 also includes a sloping surface that engages sloping surfaces of the teeth 608 so that, when the disk 602 is rotated in a second, opposite direction, the finger 604 does not engage the teeth 608 of the gear 610 so that the gear and the lead screw 202 are not rotated. Alternatively, or in addition, the gear 610 may be provided with a pawl to prevent movement in the second direction.

In the exemplary embodiment of Fig. 26, the disk 602 is movable axially along the lead screw 202, away from and towards the gear 610, and is biased towards the gear by a resilient member 612, which in turn is anchored by a stopper 614 that is immovable axially with respect to the lead screw 202. Alternatively, the disk 602 may be fixed axially with respect to the lead screw 202 and relative to the gear 262, and the finger 604 may be resilient such that the resiliency of the finger provides sufficient biasing force to engage the teeth in the first direction while leaving the finger flexible enough to move over the teeth without driving the gear in the second direction.

A shown most clearly in Figure 27, the disk 602 is rotated in first and second directions by the reciprocal actions of linear actuator having a shape memory element 646 and a return element 648 acting on a lever arm 606 of the disk 602. The return element comprises a helical compression spring 648. The disk 602 is preferably made from a stamped piece of metal or other resilient material wherein the finger 604 is formed by making cuts in the material of the disk 602 and bending the cut portions of the disk 602 in the desired direction to form the finger 604. As shown in Figure 27 there may be more than one finger 604 formed in the disk 602.

The dispenser 700 shown in Fig. 28 is similar to the dispenser 600 shown in Figs. 26 and 27, except that the teeth 263 of the gear 262 are on the outer circumference of the gear 262. A disk 702 of the dispenser 700 has a finger 704 that extends from its outer circumference to engage the teeth 263 of the gear. The disk 702 shown in Figure 28 is also, preferably, a stamped piece of metal or other sufficiently resilient material, such that the finger 704 can be bent into position and provide sufficient resilience to engage the teeth 263 on the outer circumference of the gear 262.

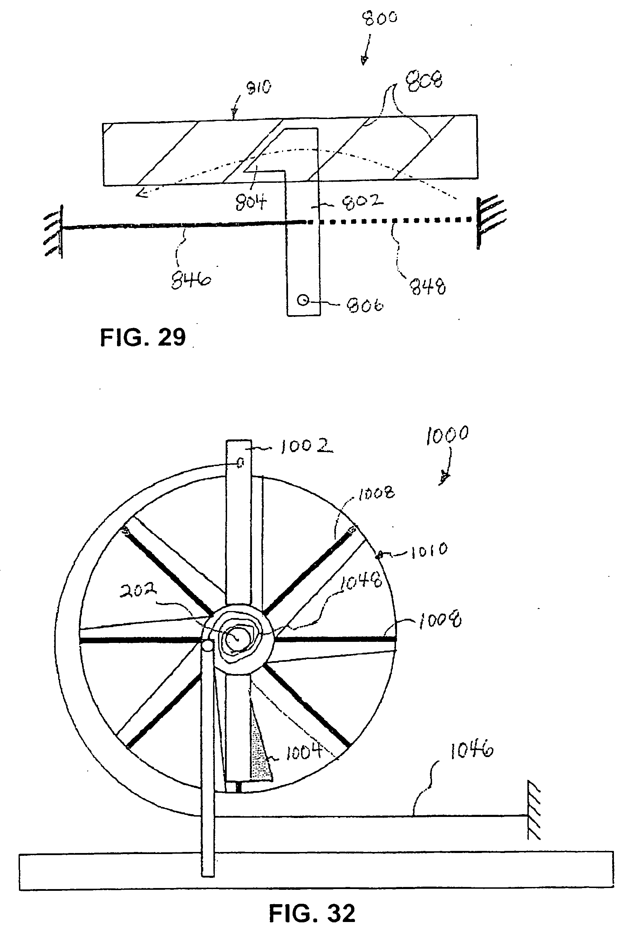

The dispenser 800 shown in Fig. 29 includes a linear actuator, preferably a shape memory element 846 and a return element 848, and an arm 802 that is pivotally mounted at a pivot point 806 that is fixed with respect to a gear 810 of the dispenser. The shape memory element 846 and the return element 848 cause the arm 802 to alternatively pivot in opposing directions about the pivot point 806. The return element comprises a helical tension spring 848. A finger 804 of the arm 802 is shaped and adapted to engage teeth 808 on the outer circumference of the gear 810. The mating edges of the teeth 808 and the finger 804 are parallel and are at an angle relative to an axis of the gear 810 so that, as the arm 802 moves through its arc of travel, the mating surfaces of the finger 804 and the teeth 808 remain substantially fully engaged.

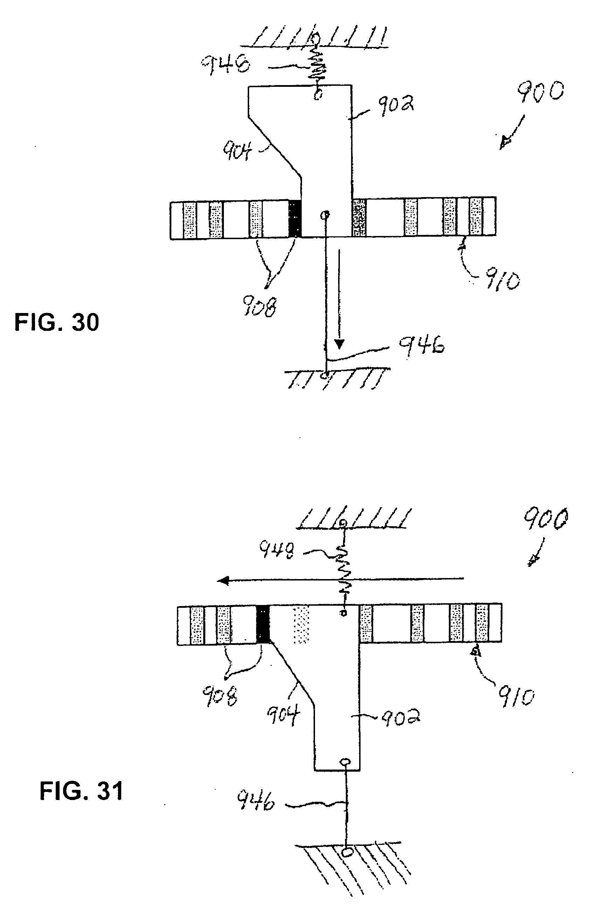

The dispenser 900 shown in Figs. 30 and 31 also includes a linear actuator in the form of a shape memory element 946 and a return element 948. The shape memory element 946 and the return element 948 move a cam 902 between a first position, shown in Fig. 30, and a second position shown in Fig. 31. The return element comprises a helical compression spring 948. The cam 902 is biased into engagement with teeth 908 of a gear 910 such that, when the cam 902 is moved from the first position shown in Fig. 30 to the second position shown in Fig. 31, a cam surface 904 of the cam 902 engages teeth 908 of the gear 910 so that the gear 910 is rotated (counter-clockwise as shown by the arrow in Fig. 31). The cam 902 is arranged and shaped such that, when the cam 902 is moved from the second position shown in Fig. 31 to the first position shown in Fig. 30, the cam surface 904 does not engage the teeth 908 of the gear 910 and the gear 910 is not rotated.

In Figure 32, the dispenser 1000 includes an arm 1002 instead of a disk, that is mounted rotatably and coaxially mounted for independent rotation on the lead screw 202. The arm 1002 has at least one finger 1004 that engages teeth 1008 extending outwardly from a face of a gear 1010. The gear 1010 is mounted on the lead screw 202 such that rotation of the gear 1010 causes rotation of the lead screw 202. The dispenser 1000 also includes a linear actuator in the form of a shape memory element 1046 and a return element that comprises a clock spring 1048 wound around the lead screw 202 such that the spring acts in opposition to the shape memory element 1046.

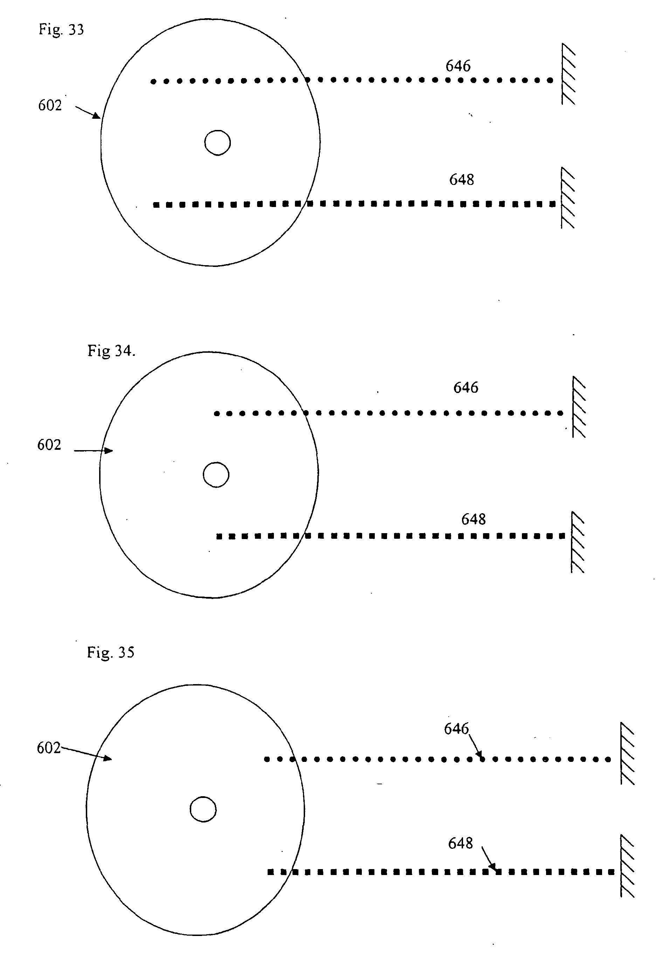

Figs. 33-35 show three alternative embodiments of attachment points of the shape memory element 646 and the return spring 648 to the disk 602 providing different mechanical advantage to the shape memory element 646 and return spring 648, respectively. In Fig. 33, the shape memory element has mechanical advantage over the return spring. In Fig. 35, the return spring has mechanical advantage over the shape memory element. In Fig. 34, the shape memory element and return spring are mechanically balanced. In general, it is preferable to give the mechanical advantage to the shape memory element. The optimum positions, and corresponding mechanical advantage, depends upon the specific properties of the shape memory element.

As illustrated by the above described exemplary embodiments, the present invention generally provides a device for delivering fluid, such as insulin for example, to a patient. The device includes an exit port assembly, a syringe-like reservoir including a side wall extending towards an outlet connected to the exit port assembly. A threaded lead screw is received in the reservoir and a plunger has an outer periphery linearly slideable along the side wall of the reservoir and an inner periphery threadedly received on the lead screw. The plunger is non-rotatable with respect to the side wall such that rotating the lead screw causes the plunger to advance within the reservoir and force fluid through the outlet. The device also includes a dispenser having a shape memory element, and a changeable length of the shape memory element decreasing from an uncharged length to a charged length causes rotation of the lead screw.

## Claims

1. A device (10) for delivering fluid to a patient, comprising:
a. a reservoir (30) including a side wall (32) extending towards an outlet (36);
b. a plunger (204) received in the reservoir (30);
c. a threaded lead screw (202) coupled to the plunger (204);
d. a dispenser including a linear actuator and a transmission that couples the linear actuator to the lead screw (202) and enables the linear actuator to rotate the lead screw (202), the transmission including,
i. a frictional engagement surface (214) coupled to the lead screw (202) such that the frictional engagement surface (214) is immovable relative to the lead screw (202);
ii. engagement means for selectively engaging the frictional engagement surface such that the engagement means engages the frictional engagement surface when the engagement means is moved in a first direction, but does not engage the frictional engagement surface when the engagement means is moved in a second direction.

2. The device of claim 1, wherein the frictional engagement surface is a gear (214) mounted coaxially with the lead screw (202).

3. The device of claim 2, wherein the gear (214) has teeth extending formed on a face thereof.

4. A device (10) for delivering fluid to a patient, comprising:
a. a reservoir (600) including a side wall (32) extending towards an outlet (602);
b. a plunger (608) received in the reservoir;
c. an assembly comprising a threaded lead screw (606) coupled to the plunger and a nut threaded onto the lead screw (606) and mounted in a fixed position relative to the lead screw (606) such that rotation of the nut moves the lead screw (606) through the nut;
d. a dispenser including a linear actuator and a transmission that couples the linear actuator to the nut and enables the linear actuator to rotate the nut, the transmission including,
i. a frictional engagement surface coupled to the nut such that the frictional engagement surface is immovable relative to the nut;
ii. engagement means for selectively engaging the frictional engagement surface such that the engagement means engages the frictional engagement surface when the engagement means is moved in a first direction, but does not engage the frictional engagement surface when the engagement means is moved in a second direction.

5. The device of claim 4, wherein the frictional engagement surface is a surface of the nut.

6. The device of claim 5, wherein the frictional engagement surface is a gear having teeth formed in the nut.

7. The device of claim 6, wherein the teeth of the gear are formed on a side of the nut.

8. The device of claim 3 or 7, wherein the engagement means comprises a substantially planar member mounted coaxially with the lead screw and adjacent to the teeth of the gear, the planar member having a flexible projecting member that projects towards the teeth, such that the projecting member engages the teeth when the planar member is rotated in a first direction and the projecting member does not engage the teeth when the planar member is rotated in a second direction.

9. The device of claim 2 or 6, wherein the teeth are formed on an outer circumferential edge of the gear.

10. The device of claim 9, wherein the engagement means is a cam mounted for linear movement substantially parallel to the axis of the gear and the cam includes a cam surface that is shaped and arranged such that, when the cam is moved from the first position to the second position, the cam surface engages a tooth and causes the gear to rotate.

11. The device of claim 6 or 9, wherein the engagement means is a hook mounted for linear movement substantially perpendicular to the axis of the gear and the hook is shaped and arranged such that, when the hook is moved from the first position to the second position, the hook engages a tooth and causes the gear to rotate.

12. The device of claim 1 or 4, wherein the linear actuator includes a shape memory element (646).

13. The device of claim 1 or 4, wherein the linear actuator includes a return element (648).

14. The device of claim 13, wherein the return element comprises a wound clock spring.

15. The device of claim 13, wherein the return element is a helical compression spring.

16. The device of claim 13, wherein the return element is a helical tension spring.

17. The device of claim 8, wherein the substantially planar member of the engagement means is formed from a single piece of material.

18. The device of claim 17, wherein the substantially planar member of the engagement means is formed form a single piece of metal.

19. The device of claim 1 or 4 wherein the second direction is opposite the first direction.

20. The device of claim 1 or 4, wherein the linear actuator includes a return element (648) and a shape memory element (646), and wherein a changeable length of the shape memory element (646) decreasing from an uncharged length to a charged length resets the return element.

## Patentansprüche

1. Eine Vorrichtung (10) zum Zuführen von Flüssigkeiten zu einem Patienten, bestehend aus:
a) einem Behälter (30) mit einer Seitenwand (32), die sich zu einem Ausgang (36) hin erstreckt;
b) einem im Behälter (30) angeordneten Kolben (204);
c) einer mit dem Kolben (204) gekoppelten Gewindeleitspindel (202);
d) einem Spender mit einem linearen Aktuator und einem Getriebe, das den linearen Aktuator mit der Leitspindel (202) verbindet und den linearen Aktuator befähigt, die Leitspindel (202) zu rotieren, wobei das Getriebe die folgenden Elemente umfasst:
i) eine mit der Leitspindel (202) so gekoppelten Reibschlussfläche (214), dass die Reibschlussfläche (214) relativ zur Leitspindel (202) unbeweglich ist;
ii) ein Eingreifmittel für das selektive Eingreifen in die Reibschlussfläche, so dass das Eingreifmittel in die Reibschlussfläche eingreift, wenn das Eingreifmittel in eine erste Richtung bewegt wird, aber nicht in die Reibschlussfläche eingreift, wenn das Eingreifmittel in eine zweite Richtung bewegt wird.

2. Die Vorrichtung nach Anspruch 1, wobei es sich bei der Reibschlussfläche um ein Zahnrad (214) handelt, das koaxial mit der Leitspindel (202) verbunden ist.

3. Die Vorrichtung nach Anspruch 2, wobei das Zahnrad (214) auf einer Außenfläche angeordnete Zähne aufweist.

4. Eine Vorrichtung (10) zum Zuführen von Flüssigkeiten zu einem Patienten, bestehend aus:
a) einem Behälter (600) mit einer Seitenwand (32), die sich zu einem Ausgang (602) hin erstreckt;
b) einem im Behälter angeordneten Kolben (608);
c) einer Baugruppe bestehend aus einer mit dem Kolben gekoppelten Gewindeleitspindel (606) und einer auf der Leitspindel (606) aufgeschraubten und relativ zur Leitspindel (606) fest positionierten Gewindemutter, so dass die Rotation der Gewindemutter die Rotation der Leitspindel (606) durch die Gewindemutter bewirkt;
d) einem Spender mit einem linearen Aktuator und einem Getriebe, das den linearen Aktuator mit der Gewindemutter verbindet und den linearen Aktuator befähigt, die Gewindemutter zu rotieren, wobei das Getriebe die folgenden Elemente umfasst:
i) eine mit der Gewindemutter so gekoppelten Reibschlussfläche (214), dass die Reibschlussfläche (214) relativ zur Gewindemutter unbeweglich ist;
ii) ein Eingreifmittel für das selektive Eingreifen in die Reibschlussfläche, so dass das Eingreifmittel in die Reibschlussfläche eingreift, wenn das Eingreifmittel in eine erste Richtung bewegt wird, aber nicht in die Reibschlussfläche eingreift, wenn das Eingreifmittel in eine zweite Richtung bewegt wird.

5. Die Vorrichtung nach Anspruch 4, wobei es sich bei der Reibschlussfläche um eine Fläche der Gewindemutter handelt.

6. Die Vorrichtung nach Anspruch 5, wobei es sich bei der Reibschlussfläche um ein Zahnrad mit Zähnen in der Gewindemutter handelt.

7. Die Vorrichtung nach Anspruch 6, wobei die Zähne des Zahnrads an einer Seite der Gewindemutter angeordnet sind.

8. Die Vorrichtung nach Anspruch 3 oder 7, wobei das Eingreifmittel ein im Wesentlichen ebenflächiges, koaxial mit der Leitspindel und neben den Zähnen des Zahnrads montiertes Element umfasst, das über ein flexibles projizierendes Teil verfügt, das sich so zu den Zähnen hin erstreckt, dass das projizierende Teil in die Zähne eingreift, wenn das ebenflächige Element in eine erste Richtung gedreht wird, und das projizierende Teil nicht in die Zähne eingreift, wenn das ebenflächige Element in eine zweite Richtung gedreht wird.

9. Die Vorrichtung nach Anspruch 2 oder 6, wobei die Zähne an einem äußeren Rand des Zahnrads angeordnet sind.

10. Die Vorrichtung nach Anspruch 9, wobei es sich bei dem Eingreifmittel um einen Nocken handelt, der für lineare Bewegungen im Wesentlichen parallel zur Achse des Zahnrads angeordnet ist, und der Nocken eine Nockenfläche aufweist, die so geformt und angeordnet ist, dass die Nockenfläche am Zahnrad einrastet, wenn der Nocken von einer ersten Position in eine zweite Position bewegt wird, und dadurch bewirkt, dass sich das Zahnrad dreht.

11. Die Vorrichtung nach Anspruch 6 oder 9, wobei es sich bei dem Eingreifmittel um einen Haken handelt, der für lineare Bewegungen im Wesentlichen im rechten Winkel zur Achse des Zahnrads angeordnet ist, und der Haken so geformt und angeordnet ist, dass der Haken sich an einem Zahn verhakt, wenn der Haken von einer ersten Position in eine zweite Position bewegt wird, und dadurch bewirkt, dass sich das Zahnrad dreht.

12. Die Vorrichtung nach Anspruch 1 oder 4, wobei der lineare Aktuator ein Formgedächtnis-Element (646) enthält.

13. Die Vorrichtung nach Anspruch 1 oder 4, wobei der lineare Aktuator ein Rückstellelement (648) enthält.

14. Die Vorrichtung nach Anspruch 13, wobei das Rückstellelement eine Wickelfeder umfasst.

15. Die Vorrichtung nach Anspruch 13, wobei es sich bei dem Rückstellelement um eine schraubenförmige Druckfeder handelt.

16. Die Vorrichtung nach Anspruch 13, wobei es sich bei dem Rückstellelement um eine schraubenförmige Zugfeder handelt.

17. Die Vorrichtung nach Anspruch 8, wobei das im Wesentlichen ebenflächige Element des Eingreifmittels aus einem einzigen Materialstück geformt ist.

18. Die Vorrichtung nach Anspruch 17, wobei das im Wesentlichen ebenflächige Element der Kupplung aus einem einzigen Metallstück geformt ist.

19. Die Vorrichtung nach Anspruch 1 oder 4, wobei die zweite Richtung entgegengesetzt zur ersten Richtung verläuft.

20. Die Vorrichtung nach Anspruch 1 oder 4, wobei der lineare Aktuator ein Rückstellelement (648) und ein Formgedächtnis-Element (646) umfasst, und wobei die Veränderung einer veränderbaren Länge des Formgedächtnis-Elements (646) von einer unbelasteten Länge zu einer belasteten Länge die Rückstellung des Rückstellelements bewirkt.

## Revendications

1. Dispositif (10) permettant d'administrer un fluide à un patient, comprenant :
a. un réservoir (30) comportant une paroi latérale (32) s'étendant vers une sortie (36) ;
b. un piston (204) logé dans le réservoir (30) ;
c. une vis-mère filetée (202) couplée au piston (204) ;
d. un distributeur comportant un actionneur linéaire et une transmission couplant l'actionneur linéaire à la vis-mère (202) et permettant à l'actionneur linéaire de tourner la vis-mère (202), la transmission comportant :
i. une surface de contact par frottement (214) couplée à la vis-mère (202) de telle sorte que la surface de contact par frottement (214) ne peut être déplacée par rapport à la vis-mère (202) ;
ii. un moyen de prise permettant de mettre en prise de manière sélective la surface de contact par frottement de telle sorte que le moyen de prise entre en prise avec la surface de contact par frottement lorsque le moyen de prise est déplacé dans une première direction, mais n'entre pas en prise avec la surface de contact par frottement lorsque le moyen de prise est déplacé dans une deuxième direction.

2. Dispositif selon la revendication 1, dans lequel la surface de contact par frottement est un engrenage (214) installé de manière coaxiale à la vis-mère (202).

3. Dispositif selon la revendication 2, dans lequel l'engrenage (214) est muni de dents en saillie, formées sur une face de celui-ci.

4. Dispositif (10) permettant d'administrer un fluide à un patient, comprenant :
a. un réservoir (600) comportant une paroi latérale (32) s'étendant vers une sortie (602) ;
b. un piston (608) logé dans le réservoir ;
c. un ensemble comportant une vis-mère filetée (606) couplée au piston, et un écrou fileté sur la vis-mère (606) et fixé en position par rapport à la vis-mère (606) de telle sorte que la rotation de l'écrou déplace la vis-mère (606) à travers l'écrou ;
d. un distributeur comportant un actionneur linéaire et une transmission couplant l'actionneur linéaire à l'écrou et permettant à l'actionneur linéaire de tourner l'écrou, la transmission comportant :
i. une surface de contact par frottement couplée à l'écrou de telle sorte que la surface de contact par frottement ne peut être déplacée par rapport à l'écrou ;
ii. un moyen de prise permettant de mettre en prise de manière sélective la surface de contact par frottement de telle sorte que le moyen de prise entre en prise avec la surface de contact par frottement lorsque le moyen de prise est déplacé dans une première direction, mais n'entre pas en prise avec la surface de contact par frottement lorsque le moyen de prise est déplacé dans une deuxième direction.

5. Dispositif selon la revendication 4, dans lequel la surface de contact par frottement est une surface de l'écrou.

6. Dispositif selon la revendication 5, dans lequel la surface de contact par frottement est un engrenage denté formé dans l'écrou.

7. Dispositif selon la revendication 6, dans lequel les dents de l'engrenage sont formées sur un côté de l'écrou.

8. Dispositif selon la revendication 3 ou 7, dans lequel le moyen de prise comprend un élément essentiellement plan, installé de manière coaxiale à la vis-mère et adjacent aux dents de l'engrenage, l'élément plan étant muni d'un élément flexible saillant faisant saillie vers les dents, de telle sorte que l'élément saillant entre en prise avec les dents lorsque l'élément plan est tourné dans une première direction, et que l'élément saillant n'entre pas en prise avec les dents lorsque l'élément plan est tourné dans une deuxième direction.

9. Dispositif selon la revendication 2 ou 6, dans lequel les dents sont formées sur un bord périphérique extérieur de l'engrenage.

10. Dispositif selon la revendication 9, dans lequel le moyen de prise est une came installée en mouvement linéaire essentiellement parallèle à l'axe de l'engrenage, et la came comporte une surface de came formée et disposée de telle sorte que, lorsque la came passe de la première position à la deuxième position, la surface de came entre en prise avec une dent et entraîne la rotation de l'engrenage.

11. Dispositif selon la revendication 6 ou 9, dans lequel le moyen de prise est un crochet installé en mouvement linéaire essentiellement perpendiculaire à l'axe de l'engrenage, et le crochet est formé et disposé de telle sorte que, lorsque le crochet passe de la première position à la deuxième position, le crochet entre en prise avec une dent et entraîne la rotation de l'engrenage.

12. Dispositif selon la revendication 1 ou 4, dans lequel l'actionneur linéaire comporte un élément à mémoire de forme (646).

13. Dispositif selon la revendication 1 ou 4, dans lequel l'actionneur linéaire comporte un élément de rappel (648).

14. Dispositif selon la revendication 13, dans lequel l'élément de rappel comporte un ressort d'horlogerie remonté.

15. Dispositif selon la revendication 13, dans lequel l'élément de rappel comporte un ressort de compression hélicoïdal.

16. Dispositif selon la revendication 13, dans lequel l'élément de rappel comporte un ressort de tension hélicoïdal.

17. Dispositif selon la revendication 8, dans lequel l'élément essentiellement plan du moyen de prise est formé à partir d'une seule pièce de matériau.

18. Dispositif selon la revendication 17, dans lequel l'élément essentiellement plan du moyen de prise est formé à partir d'une seule pièce métallique.

19. Dispositif selon la revendication 1 ou 4, dans lequel la deuxième direction est opposée à la première direction.

20. Dispositif selon la revendication 1 ou 4, dans lequel l'actionneur linéaire comporte un élément de rappel (648) et un élément à mémoire de forme (646), et dans lequel une longueur variable de l'élément à mémoire de forme (646), diminuant d'une longueur non chargée à une longueur chargée, réamorce l'élément de rappel.
